# EUROPEAN PATENT SPECIFICATION

(11) **EP 0 524 495 B1**
(45) Date of publication and mention of the grant of the patent: **09.10.1996**
(21) Application number: 92111663.8
(22) Date of filing: 09.07.1992
(51) Int. Cl.: C07D 309/32, C07C 69/675

(54) **Process for the preparation of pyranones and pyrandiones**
Verfahren zur Herstellung von Pyranonen und Pyrandionen
Procédé pour la préparation des pyranones et pyrandiones

(30) Priority: 23.07.1991 US 734408; 12.03.1992 US 849908
(43) Date of publication of application: 27.01.1993
(73) Proprietor: F. HOFFMANN-LA ROCHE & CO. Aktiengesellschaft, 4002 Basel (CH)
(72) Inventor: Landi, John Joseph, Jr., East Stroudsburg, Pennsylvania 18301 (US); Ramig, Keith Marriott, Orange, New Jersey 07050 (US)
(74) Representative: Mahé, Jean

(56) References cited:
- JOURNAL OF ORGANIC CHEMISTRY, vol. 53, no. 6, 1988, Washington,DC, US, pages 1218 - 1221; P. BARBIER ET AL.: 'Synthesis of tetrahydrolipstatin and tetrahydroesterastatin, compounds with a beta-lactone moiety. Stereoselective hydrogenation of a beta-keto delta-lactone and conversion of the delta-lactone into a beta-lactone'

## Description

The invention relates to a novel process for the preparation of pyranones and pyrandiones, namely of a tautomeric mixture of compounds of the formulas

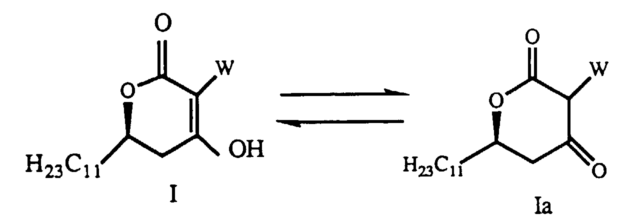

The compounds of formulas I and Ia are known precursors for the preparation of tetrahydrolipstatin, as described for example in J.Org. Chem., 53, 1988, 1218-1221.

As used herein, the term "leaving group" denotes a group subject to nucleophilic substitution, such as, for example, halogen, preferably chlorine; unsubstituted or substituted alkoxy, other than tert butyloxy and tert amyloxy, preferably methoxy; unsubstituted or substituted aryloxy, unsubstituted or substituted acyloxy, alkoxy carbonyloxy and amino carbonyloxy.

The term "reducible group" denotes a group subject to reductive removal by low valent metals, such as, for example, halogen, preferably bromine; alkylsulfide, arylsulfide, alkylselenide and arylselenide.

The term "alkyl", alone or in combination, denotes a straight-chain or branched chain alkyl group containing 1 to 7, preferably 1 to 4, carbon atoms, especially preferred is methyl. Examples of alkoxy groups are methoxy, ethoxy, propoxy, isopropoxy and butoxy.

The term "aryl", alone or in combination, denotes a monocyclic or polycyclic aromatic group, preferably a monocyclic or bicyclic group, for example, phenyl or naphthyl. Exemplary of aryl in combination with alkyl denotes, for example, benzyl.

The term "acyloxy", alone or in combination denotes alkyl or aryl attached to a carboxyl group, such as, for example, acetoxy and benzoyloxy.

The term "substituted alkoxy" denotes an alkoxy group, as defined earlier, which is substituted with, for example, one or more substituents selected from alkoxy, alkoxyalkoxy, dialkylamino alkoxy, fluorine, and aryl.

The term "substituted aryloxy" denotes an aryloxy group as defined earlier which is substituted with one or more substituents, for example, substituents selected from 2-nitro, 4-nitro, 2-alkoxycarbonyl, 4-alkoxycarbonyl, 2-trifluoromethyl, 3-trifluoromethyl, 4-trifluoromethyl, 2-cyano, 3-cyano, 4-cyano, 2-chloro, 3-chloro, 4-chloro, pentachloro, and pentafluoro.

The term "substituted amine" denotes an amine group which is substituted, for example, with one or more substituents selected from alkyl, alkoxyalkyl, aryl, and alkylaryl.

The term "substituted arylalkoxy" denotes an arylalkoxy group as defined above which is substituted with one or more substituents, for example, the substituents mentioned above in connection with "substituted aryloxy".

The term "metal" when used alone denotes a metal prone to forming stable alkoxides, such as, for example, lithium, sodium, potassium, magnesium and calcium.

The term "low valent metal" denotes metals, such as, for example, Zn, Li, Na, K and the like, preferably Zn, and alloys of Zn, such as Zn[Cu], Zn[Ag], Zn[Hg]. Especially preferred is zinc in the presence of chlorotrimethylsilane.

In accordance with the invention,the reaction step a) is carried out in a conventional aprotic organic solvent, for example, hexane or ether, preferably hexane. When R¹ is a metal the preferred solvent is THF. When Z is hydroxy the preferred solvent is methylene chloride. The reaction is preferably carried out in the presence of a base such as, pyridine, preferably under an inert atmosphere, for example, nitrogen, preferably at a temperature in the range of from about 0°C to 25°C. The compound IV can be purified, for example, by radial chromatography eluting with CH₂Cl₂.

The reaction step b) is carried out with one of the low valent metals mentioned above, in a conventional aprotic organic solvent, preferably ether, at preferably reflux temperature. The tautomeric mixture of compounds of the formulas I and Ia is isolated, for example, by extraction with organic solvents, such as ether or ethyl acetate, preferably ether, and purified, for example, by trituration with hexane.

The starting material of formula II wherein R¹ is hydrogen, can be prepared by known methods as illustrated below.

When R is alkoxy, substituted alkoxy, substituted amine, the compound of formula II can be prepared by asymmetric hydrogenation of the corresponding β-keto-ester, which are known compounds or can be prepared by known methods. For example, the β-keto-ester can be prepared by a two step process involving condensation of lauroyl chloride with Meldrum's acid to form the compound of the formula 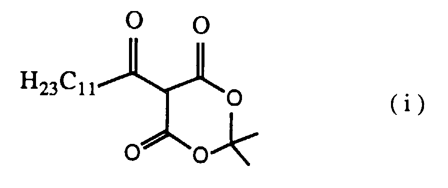 which can be treated with the desired alcohol or amine.

When R is benzyloxy, aryloxy or substituted aryloxy, the compound of formula II can be prepared by desilylation of a β-silyloxycarboxylic ester of the formula 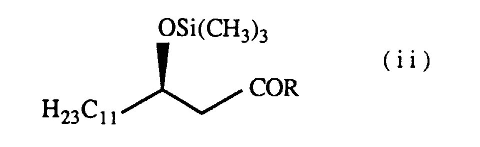 which is formed by esterification of β-silyloxy carboxylic acid of the formula 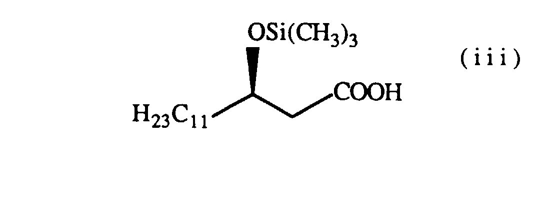 prepared by silylation of R-3-hydroxytetradecanoic acid of the formula

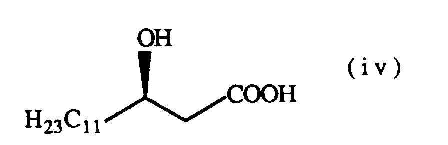

The starting materials of formula II wherein R¹ is metal are novel and can be prepared by treating a compound of formula 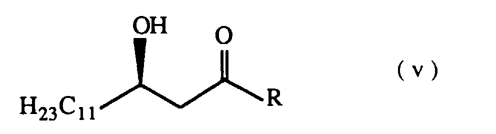 wherein R is alkoxy, substituted alkoxy, substituted amine, unsubstituted or substituted arylalkoxy, with the appropriate metal hydride in an aprotic organic solvent.

The compounds of formula III are known compounds or can be prepared according to known procedures, for example, as in Synthetic Reagents Vol. 1, 321-357, Wiley, NY (1974); or in Synthesis, 1982, 684.

In a preferred embodiment, in the compound of formula II, R is alkoxy, preferably, methoxy, R¹ is hydrogen and in the compound of formula III, X is halogen, preferably, bromine, Y is hydrogen, and Z is halogen, preferably chlorine.

The examples which follow further illustrate the invention.

### Example 1

To a solution of 20 g 2-bromooctanoic acid in 100 ml toluene was added 13.2 ml thionyl chloride. The solution was heated at reflux under nitrogen for 3 hours. The excess thionyl chloride and toluene were distilled at atmospheric pressure under N₂. The residue was purified by distillation (bp = 60-65° C/13.3 Pa [0.1 mm Hg]) and 13.8g (64%) of 2-bromooctanoyl chloride was isolated, IR (CHCl₃) 1788 (C=O) cm⁻¹

### Example 2

To a stirred slurry of 5.18 g methyl (R)-3-hydroxytetradecanoate and 5.03 g 2-bromooctanoyl chloride in 100 ml hexane at 0° C under N₂ was added 1.67 ml pyridine. After stirring the mixture was poured into a mixture of 200 ml H₂O/100 ml ether. This mixture was shaken and the layers separated. The aqueous layer was extracted with ether. The ether layers were washed with brine, and H₂O. The ether was dried and removed by evaporation. The product was dissolved in CH₂Cl₂ and filtered through silica gel, washing with CH₂Cl₂.Evaporation gave 8.10 g (87%) (R)-3-[(2-bromo-1-oxooctyl)oxy]tetradecanoic acid methyl ester, which was found to be suitable for use in the cyclization reaction producing the lactone of Example 5. Analytically pure samples could be prepared by subjecting the oil to radial chromatography, eluting with CH₂Cl₂. The purified product showed two diastereomers,

¹H NMR (CDCl₃) δ 0.85-0.95 (m,6H,CH₃), 1.20-1.50 (m,26H,CH₂), 1.58-1.72(m,2H,CH₂), 1.89-2.11(m,2H,CH₂), 2.53-2.69(m,2H,CH₂CO), 3.67(s, 0.43H, OMe of one diastereomer), 3.68(s,0.57H, OMe of the other diastereomer), 4.17(t, J=7.43 Hz, 1H, CHBr), 5.28 (m,1H,HCO).

### Example 3

The following were added sequentially to 200 ml CH₂Cl₂ under N₂: 10.0 g 2-bromooctanoic acid, 9.30 g methyl(R)-3-hydroxytetradecanoate, 7.43 g N,N'-dicyclohexylcarbodiimide, and 0.508 g 4-dimethylaminopyridine. The mixture was stirred for 1 hour, followed by removal of the solvent by evaporation. The resulting solid was stirred with hexane for 5 minutes, followed by filtration and washing with hexane. The filtrate was washed with 1*M* HC1, 10% NaHCO₃ and H₂O. Drying and removal of the solvent by evaporation gave a 16.1 g mixture of oil and solid particles. This mixture was dissolved in 50% CH₂Cl₂/hexane and chromatographied on silica gel,eluting with CH₂Cl₂/hexane. Isolated was 14.2 g (85% yield based on 2-bromooctanoic acid) (R)-3-[2-bromo-1-oxooctyl)-oxy]tetradecanoic acid methyl ester.

### Example 4

98 mg sodium hydride(80% in mineral oil)was added to a solution of 764 mg methyl(R)-3-hydroxytetradecanoate in 20.0 ml THF. A solution of 732 mg 2-bromooctanoyl chloride in 10.0 ml THF was added dropwise to the mixture. After 10 minutes at 0°C, the reaction flask was removed from the cold bath for 24 hours. The mixture was poured into 10% NaHCO₃ and extraction with ether was performed. Drying and removal of the solvent by evaporation gave an oil. Purification by flash chromatography, eluting with CH₂Cl₂/hexane gave 407 mg [30% yield based on methyl (R)-3-hydroxytetradecanoate] (R)-3-[2-bromo-l-oxooctyl)oxy]-tetradecanoic acid methyl ester.

### Example 5

A mixture of 744 mg zinc and 1.03 g bromodiester from example 2 in 10 ml 20% v:v Et₂O in chlorotrimethylsilane was heated at reflux under N₂ with stirring for 20 minutes. The mixture was cooled to room temperature, filtered and washed with 10 ml Et₂O. The filtrate was cooled in an ice/H₂O bath and H₂O was added with stirring. After 10 minutes, the mixture was poured into H₂O and extraction was performed with Et₂O. The organic layers were washed with brine and dried. The Et₂O was removed by evaporation. The resulting solid was slurried in hexane, then filtered and washed with hexane. The product was dried, giving 478 mg (61%) (R)-3-hexyl-5,6-dihydro-4-hydroxy-6-undecyl-2H-pyran-2-one, mp=110-112°C, [α]$\frac{\text{20}}{\text{D}}$= 44.69° (1% in dioxane).

### Example 6

A mixture of 2.44 g (R)-3-hydroxytetradecanoic acid, 2.72 g imidazole, 3.03 g t-butyldimethylsilyl chloride, and 50 ml dichloromethane was stirred at room temperature for 22 hours. 5 ml methanol was added and the mixture was stirred at room temperature.After 15 minutes, it was concentrated to dryness. The residue was chromatographed on silica gel in chloroform. The product was eluted with 5% methanol in chloroform, giving 2.84 g (79% yield) (R)-3-t-butyldimethylsilyloxytetradecanoic acid.

### Example 7

A solution of 2.71 g (R)-3-t-butyldimethylsilyloxytetradecanoic acid in 20 ml dichloromethane was cooled with stirring to 5° C.First 0.72 g phenol, then 1.72 g N,N'-dicyclohexyl-carbodiimide were added and the mixture was allowed to warm to room temperature and stirred for 16 hours. The mixture was filtered, and the collected solids were washed with dichloromethane. The filtrate and washes were concentrated to dryness. The residue was chromatographed on silica gel in dichloromethane. The product was eluted with dichloromethane. One obtains 2.71 g, (82% yield) phenyl(R)-3-t-butyldimethylsilyloxytetradecanoate.
¹HNMR(CDCl₃) δ0.084(m,6H),0.89(m,12H),1.20-1.40(m,18H),1.56(m,2H),2.68(m,2H), 4.24(m,1H), 7.08(m,2H),7.23(m,1H),7.37 (m,2H).

### Example 8

A solution of 5.80 g phenyl(R)-3-t-butyl-dimethylsilyloxytetradecanoate, 54 ml THF, 6 ml acetic acid, and 25.26 g tetrabutylammonium fluoride trihydrate was stirred for 22.5 hours. The solution was then concentrated and to the residue was added 70 ml ethyl acetate. It was then washed with 0.1 N hydrochloric acid, with saturated sodium bicarbonate and with saturated sodium chloride and finally dried. The mixture was then filtered and concentrated. The residue was chromatographed on silica gel in hexane, giving after concentration 1.02 g (24% yield) phenyl(R)-3-hydroxytetradecanoate.
¹H NMR(CDCl₃) δ0.89(t,3H),1.27(m,16H),1.55(m,4H),2.71 (m,3H), 4.12(m,1H),7.09(d,2H),7.26(m,1H),7.39(t,3H).

### Example 9

A mixture of 1.02 g phenyl-(R)-3-hydroxy-tetradecanoate, 22 ml dichloromethane, 0.74 g 2-bromooctanoic acid, 0.66 g N,N'- dicyclohexylcarbodiimide and 0.39 g 4-dimethylaminopyridine was stirred for 1.5 hours. The solution was then concentrated to dryness. 25 ml hexane was added to the residue and the mixture was stirred for 5 minutes. The slurry was filtered and the solids were washed with hexane. The filtrates were washed with 1M HCl, 10% saturated sodium bicarbonate and water. The organic layer was dryed, then filtered and concentrated. The residue was chromatographed on silica gel in hexane. The product was eluted with 50/50 hexane and dichloromethane, giving after concentration 0.98 g, (58% yield) of phenyl-(R)-3-(2-bromo-1-oxooctyloxy)tetradecanoate;
¹H NMR(CDCl₃) δ0.88(m,3H),1.26(m,26H),1.40(m,2H),1.74 (m,2H),1.97(m,1H),2.03(m,1H),2.84(m,2H),4.20(m,1H), 5.41(m,1H),7.09(m,2H),7.23(m,1H),7.37(t,2H).

### Example 10

Under N₂ a mixture of 336 mg zinc and 510 mg of the product of Example 9 in 5.0 ml 20% v:v Et₂O:chlorotrimethylsilane was heated at reflux with stirring for 20 minutes. The mixture was cooled to room temperature, then filtered and washed with Et₂O. The filtrate was cooled in an ice/HO bath and H₂O was added with stirring. After 10 min, the mixture was poured into H₂O and extraction with Et₂O was performed. The organic layers were washed with brine and dried. The Et₂O was removed by evaporation. The resulting solid was stirred with hexane and then filtered. The product was washed with hexane and then dried,giving 235 mg (67%) (R)-3-hexyl-5,6-dihydro-4-hydroxy-6-undecyl-2H-pyran-2-one, mp = 109-111°C; [α]$\frac{\text{20}}{\text{D}}$ -43.5° (1% in dioxane).

### Example 11

To a stirred slurry of 1.29 g methyl (R)-3-hydroxytetradecanoate and 0.45 ml bromoacetyl bromide in 25 ml hexane at 0°C was added unter N₂ 0.42 ml pyridine. The mixture was filtered and washed with ether. The filtrate was washed with H₂0 and with 10% NaHCO₃. The ether was dried and removed by evaporation, giving 1.70 g (90%) (R)-3- [(bromoacetyl)oxy]tetradecanoic acid methyl ester;
¹H NMR(CDCl₃)δ0.864-0.897(t,J=6.67 Hz,3H,CH₃), 1.19-1.40(m,18H,CH₂), 1.58-1.71(m,2H,CH₂)2.55-2.68(m,2H, CH₂-CO₂Me),3.69(s,3H,O-CH₃), 3.80(s,2H,CH₂-Br), 5.24-5.31(m, 1H, CH-O).

### Example 12

Under N₂ a mixture of 659 mg zinc and 755 mg (R)-3-[(bromoacetyl)oxy]tetradecanoic acid methyl ester in 10 ml 20% v:v Et₂O:chlorotrimethylsilane was heated at reflux with stirring for 20 min. The mixture was cooled to room temperature and then filtered and washed with Et₂O. The filtrate was cooled in an ice/H₂O bath and H₂O was added with stirring. Then, the mixture was poured into H₂O and extraction with Et₂O was performed. The organic layers were washed with brine and then dried. The Et₂O was removed by evaporation. The resulting solid was slurried in hexane for 30 min, followed by filtration. The product was dried, giving 120 mg (22%) (R)-5,6-dihydro-4-hydroxy-6-undecyl -2H-pyran-2-one, mp= 86-7°C.
[α]$\frac{\text{20}}{\text{D}}$= -43.07°(0.98% in dioxane)

## Claims

1. A process for the preparation of a tautomeric mixture of compounds of the formulas 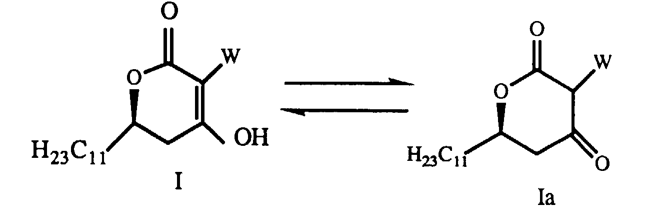 wherein W is hydrogen or C₆H₁₃,
which comprises
a) treating a compound of the formula 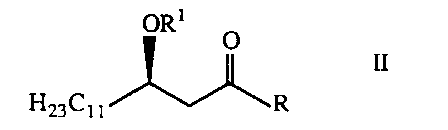 wherein R is unsubstituted or substituted alkoxy, arylalkoxy, aryloxy or amine, and R¹ is hydrogen or, when R is alkoxy, substituted alkoxy, substituted amine, unsubstituted or substituted arylalkoxy, R¹ can also be a metal, with a compound of the formula 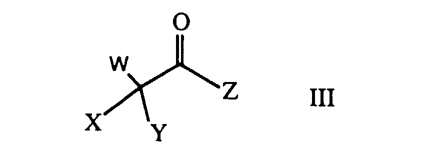 wherein W is defined above, one of X and Y is a reducible group and the other is hydrogen or a reducible group; and Z is hydroxy or a leaving group, in an aprotic organic solvent to yield a compound of the formula 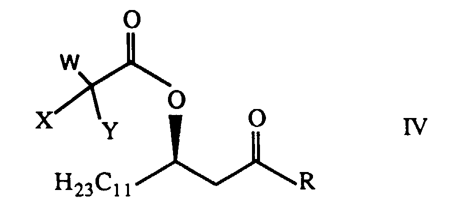 wherein R, W, X and Y are as defined above, and
b) treating a compound of formula IV with a low valent metal in an aprotic organic solvent to yield the tautomeric mixture of compounds of formulas I and Ia, and if desired, recovering the tautomeric mixture of compounds of formulas I and Ia.

2. The process of claim 1, wherein R is alkoxy, particularly methoxy, and wherein R¹ is hydrogen.

3. The process of claim 1, wherein one of X and Y is halogen, alkyl sulfide, aryl sulfide, alkyl selenide or aryl selenide, and the other is hydrogen, and Z is hydroxy, halogen, unsubstituted or substituted alkoxy, aryloxy, acyloxy, alkoxy-carbonyloxy or amino carbonyloxy.

4. The process of claim 3, wherein one of and X and Y, and Z are halogen, particularly wherein X or Y is bromine and Z is chlorine.

5. The process of claim 1, wherein the low valent metal is zinc, lithium, sodium, potassium, zinc copper, zinc silver or zinc mercury, particularly wherein the low valent metal is zinc in chlorotrimethylsilane.

6. The process of claim 1, wherein the aprotic organic solvent is hexane in step a) and ether in step b).

7. The process of claim 1, wherein W is C₆H₁₃.

8. A compound of the formula 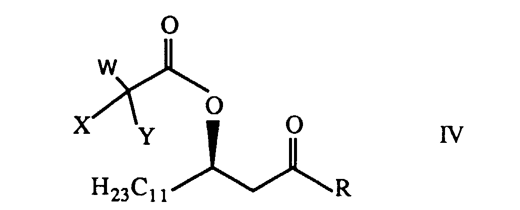 wherein one of X and Y is a reducible group and the other is hydrogen or a reducible group; W is hydrogen or C₆H₁₃ and R is unsubstituted or substituted alkoxy, aryloxy or amine.

9. The compound of claim 8, wherein one of X and Y is halogen, alkyl sulfide, aryl sulfide, alkyl selenide or aryl selenide and the other is hydrogen.

10. The compound of claim 9, wherein X or Y is halogen and R is alkoxy, particularly wherein X or Y is bromine and R is methoxy.

11. The compound of claim 8, wherein W is C₆H₁₃.

## Patentansprüche

1. Verfahren zur Herstellung eines tautomeren Gemisches von Verbindungen der Formeln 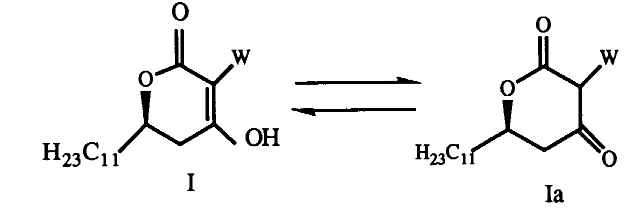 worin W Wasserstoff oder C₆H₁₃ ist,
dadurch gekennzeichnet, dass man
a) eine Verbindung der Formel 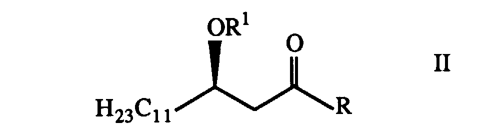 worin R unsubstituiertes oder substituiertes Alkoxy, Arylalkoxy, Aryloxy oder Amino und R¹ Wasserstoff ist oder, falls R Alkoxy, substituiertes Alkoxy, substituiertes Amino, unsubstituiertes oder substituiertes arylalkoxy ist, R¹ auch ein Metall sein kann, mit einer Verbindung der Formel 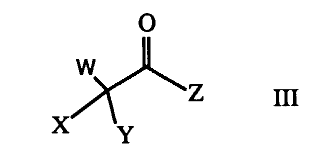 worin W wie weiter oben definiert ist, eins von X und Y eine reduzierbare Gruppe und das andere Wasserstoff oder eine reduzierbare Gruppe; und Z Hydroxy oder eine Abgangsgruppe ist, in einem aprotischen organischen Lösungsmittel behandelt, wobei man eine Verbindung der Formel 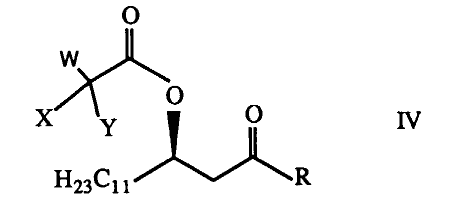 worin R, W, X und Y wie weiter oben definiert sind, erhält und
b) eine Verbindung der Formel IV mit einem Metall niedriger Valenz in einem aprotischen organischen Lösungsmittel behandelt, wobei man ein tautomeres Gemisch von Verbindungen der Formeln I und Ia erhält, und gewünschtenfalls das tautomere Gemisch der Verbindungen der Formeln I und Ia isoliert.

2. Verfahren nach Anspruch 1, worin R Alkoxy, insbesondere Methoxy, und R¹ Wasserstoff ist.

3. Verfahren nach Anspruch 1, worin eins von X und Y Halogen, Alkylsulfid, Arylsulfid, Alkylselenid oder Arylselenid, und das andere Wasserstoff, und Z Hydroxy, Halogen, unsubstituiertes oder substituiertes Alkoxy, Aryloxy, Acyloxy, Alkoxycarbonyloxy oder Aminocarbonyloxy ist.

4. Verfahren nach Anspruch 3, worin eins von X und Y, und Z Halogen sind, insbesondere worin X oder Y Brom und Z Chlor ist.

5. Verfahren nach Anspruch 1, worin das Metall niedriger Valenz Zink, Lithium, Natrium, Kalium, Zink-Kupfer, Zink-Silber oder Zink-Quecksilber, insbesondere worin das Metall von niedriger Valenz Zink in Chlortrimethylsilan ist.

6. Verfahren nach Anspruch 1, worin das aprotische organische Lösungsmittel in Stufe a) Hexan und in Stufe b) Ether ist.

7. Verfahren nach Anspruch 1, worin W C₆H₁₃ ist.

8. Eine Verbindung der Formel 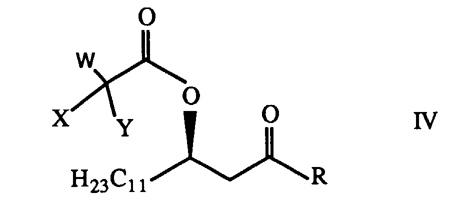 worin eins von X und Y eine reduzierbare Gruppe und das andere Wasserstoff oder eine reduzierbare Gruppe; W Wasserstoff oder C₆H₁₃ und R unsubstituiertes oder substituiertes Alkoxy, Aryloxy oder Amino ist.

9. Verbindung nach Anspruch 8, worin eins von X und Y Halogen, Alkylsulfid, Arylsulfid, Alkylselenid oder Arylselenid und das andere Wasserstoff ist.

10. Verbindung nach Anspruch 9, worin X oder Y Halogen und R Alkoxy, insbesondere worin X oder Y Brom und R Methoxy ist.

11. Verbindung nach Anspruch 8, worin W C₆H₁₃ ist.

## Revendications

1. Procédé de préparation d'un mélange tautomère de composés de formules 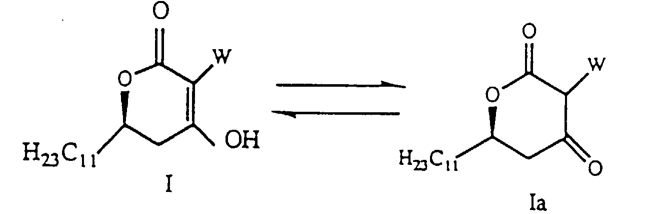 dans lesquelles W est un atome d'hydrogène ou C₆H₁₃, qui comprend les étapes consistant à
a) traiter un composé de formule 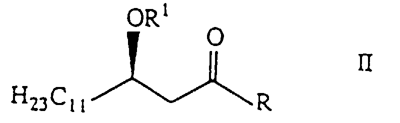 dans laquelle R est un groupe alcoxy, arylalcoxy, aryloxy ou amine, non substitué ou substitué, et R¹ est un atome d'hydrogène ou, lorsque R est un groupe alcoxy, alcoxy substitué, amine substituée, arylalcoxy non substitué ou substitué, R¹ peut aussi être un métal, avec un composé de formule 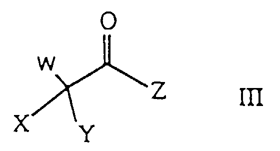 dans laquelle W est défini ci-dessus, l'un des radicaux X et Y est un groupe réductible et l'autre est un atome d'hydrogène ou un groupe réductible; et Z est un groupe hydroxy ou un groupe partant, dans un solvant organique aprotique, pour obtenir un composé de formule 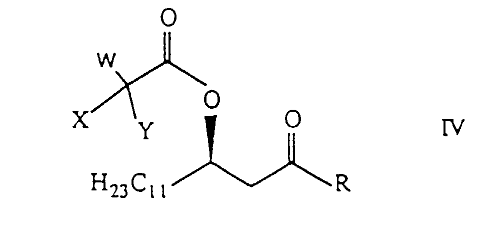 dans laquelle R, W, X et Y sont tels que définis ci-dessus, et
b) traiter un composé de formule IV avec un métal de faible valence, dans un solvant organique aprotique, pour obtenir le mélange tautomère de composés de formules I et Ia, et, le cas échéant, récupérer le mélange tautomère de composés de formules I et Ia.

2. Procédé selon la revendication 1, dans lequel R est un groupe alcoxy, en particulier méthoxy, et dans lequel R¹ est un atome d'hydrogène.

3. Procédé selon la revendication 1, dans lequel l'un des radicaux X et Y est un halogène, un sulfure d'alkyle, un sulfure d'aryle, un séléniure d'alkyle ou un séléniure d'aryle, et l'autre est un atome d'hydrogène, et Z est un groupe hydroxy, halogéno, alcoxy, aryloxy, acyloxy, alcoxycarbonyloxy ou aminocarbonyloxy, non substitué ou substitué.

4. Procédé selon la revendication 3, dans lequel l'un des radicaux X et Y, et Z, sont des atomes d'halogène, en particulier dans lequel X ou Y est un brome et Z est un chlore.

5. Procédé selon la revendication 1, dans lequel le métal à faible valence est du zinc, du lithium, du sodium, du potassium, du zinc-cuivre, du zinc-argent ou du zinc-mercure, en particulier dans lequel le métal à faible valence est du zinc dans du chlorotriméthylsilane.

6. Procédé selon la revendication 1, dans lequel le solvant organique aprotique est l'hexane dans l'étape a) et l'éther dans l'étape b).

7. Procédé selon la revendication 1, dans lequel W est C₆H₁₃.

8. Composé de formule 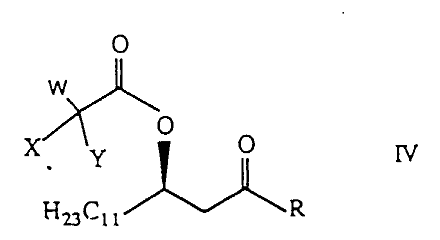 dans laquelle l'un des radicaux X et Y est un groupe réductible et l'autre est un atome d'hydrogène ou un groupe réductible; W est un atome d'hydrogène ou C₆H₁₃ et R est un groupe alcoxy, aryloxy ou amine, non substitué ou substitué.

9. Composé selon la revendication 8, dans lequel l'un des radicaux X et Y est un halogène, un sulfure d'alkyle, un sulfure d'aryle, un séléniure d'alkyle ou un séléniure d'aryle, et l'autre est un atome d'hydrogène.

10. Composé selon la revendication 9, dans lequel X ou Y est un halogène et R est un groupe alcoxy, en particulier dans lequel X ou Y est un brome et R est un groupe méthoxy.

11. Composé selon la revendication 8, dans lequel W est C₆H₁₃.
